# EUROPÄISCHE PATENTSCHRIFT

(11) **EP 1 397 082 B1**
(45) Veröffentlichungstag und Bekanntmachung des Hinweises auf die Patenterteilung: **12.04.2006**
(21) Anmeldenummer: 02748804.8
(22) Anmeldetag: 20.06.2002
(51) Int. Cl.: A61B 18/00

(54) **ELEKTROCHIRURGISCHES INSTRUMENT**
ELECTROSURGICAL INSTRUMENT
INSTRUMENT ELECTROCHIRURGICAL

(30) Priorität: 22.06.2001 DE 10129699
(43) Veröffentlichungstag der Anmeldung: 17.03.2004
(73) Patentinhaber: Erbe Elektromedizin GmbH, 72072 Tübingen (DE)
(72) Erfinder: FARIN, Günter, 72072 Tübingen (DE); SCHÄLLER, Daniel, 72074 Tübingen (DE); VOIGTLÄNDER, Mathias, 72070 Tübingen (DE)
(74) Vertreter: Bohnenberger, Johannes
(86) Internationale Anmeldenummer: PCT/EP2002/006857
(87) Internationale Veröffentlichungsnummer: WO 2003/000149

(56) Entgegenhaltungen:
- EP-A- 1 090 597
- DE-U- 29 724 247
- US-A- 6 039 736
- US-B1- 6 197 026

## Beschreibung

Die Erfindung betrifft ein elektrochirurgisches Instrument zur Koagulation biologischer Gewebe nach dem Oberbegriff des Patentanspruches 1.

Die Plasmachirurgie ist ein Verfahren der monopolaren Hochfrequenzchirurgie (HF-Chirurgie), bei welchem ein von einem Hochfrequenzgenerator (HF-Generator) generierter, hochfrequenter elektrischer Strom (HF-Strom) durch ein ionisiertes Edelgas (Plasma), beispielsweise Argon (Argon-Plasma), von einem elektrischen Pol innerhalb eines hierfür geeigneten chirurgischen Instruments auf das zu behandelnde Gewebe appliziert und von dort durch eine am Patienten applizierte sogenannte Neutralelektrode zum HF-Generator zurückgeleitet wird (G. Farin et al.: Technology of Argon Plasma Coagulation with Particular Regard to Endoscopic Applications; Endoscopic Surgery and Allied Technologies, No. 1, vol. 2, Februar 1994, 71-77). Hierdurch wird sowohl endogen durch den HF-Strom als auch exogen durch die zum Gewebe relativ höhere Temperatur des Plasmas Wärme in dieses Gewebe eingebracht, welche dessen Temperatur erhöht. In Abhängigkeit von der Temperatur werden im Gewebe verschiedene thermische Effekte verursacht, welche von Chirurgen für verschiedene therapeutische Zwecke, wie beispielsweise zur Blutstillung beziehungsweise Hämostase und/oder zur thermischen Devitalisation oder Destruktion pathologischer Gewebe genutzt werden können (K.E. Grund et al.: Argon Plasma Coagulation in Flexible Endoscopy, Endoscopic Surgery and Allied Technologies, No. 1, vol. 2, Februar 1994, 42-46).

Eine wesentliche physikalische Voraussetzung der Plasmachirurgie ist ein Edelgas, wie beispielsweise das genannte Argon oder Helium, welches zwischen dem elektrischen Pol, welcher von einer Elektrode innerhalb des Instruments gebildet wird und dem zu behandelndem Gewebe vorhanden sein muß. Edelgase sind nämlich im Vergleich zu Sauerstoff und/oder Stickstoff beziehungsweise Luft mit relativ geringen elektrischen Feldstärken ionisierbar und reagieren mit dem Gewebe nicht chemisch. Folglich wird das Gewebe auch nicht karbonisiert oder gar vaporisiert.

Innerhalb der letzten fünf Jahre hat die Plasmachirurgie, insbesondere in der flexiblen Endoskopie, ein breites Indikationsspektrum gefunden (K.E. Grund: DMW), welches jedoch unterschiedliche Anforderungen an die Applikationstechnik und die hierfür erforderlichen Instrumente, HF-Generatoren und Gasquellen stellen.

Aus der DE 198 20 240 A1 ist eine Sonde nach dem Oberbegriff des Patentanspruches 1 bekannt, die es ermöglicht, großflächige Läsionen besser als bisher zu behandeln. Besonders dann aber, wenn die Plasmachirurgie in engen Körperhöhlen bzw. Hohlorganen angewendet werden muß, kann es bei Anwendung von Instrumenten entsprechend DE 198 20 240 A1 mit nur einer seitlichen Öffnung einerseits Probleme mit der Ausrichtung dieser einen Öffnung auf das Zielgewebe, nämlich die beabsichtigt zu koagulierende Läsion, oder bei Anwendung von Instrumenten entsprechend DE 198 20 240 A1 mit mehreren am Umfang des Instruments verteilten Öffnungen andererseits Probleme mit unbeabsichtigter Koagulation anderer Gewebestellen statt dem Zielgewebe, geben. Letzteres kann es geben, wenn der Abstand des Instruments zu Geweben, welche nicht koaguliert werden sollen oder gar dürfen geringer ist als zum Zielgewebe.

Der Erfindung liegt die Aufgabe zugrunde, eine Sonde der eingangs genannten Art dahingehend weiterzubilden, daß eine verbesserte Handhabung, insbesondere im Bezug auf eine örtliche Steuerung des Plasmaverlaufes ermöglicht wird.

Diese Aufgabe wird durch ein Instrument gemäß Anspruch 1 gelöst.

Ein wesentlicher Punkt der Erfindung liegt darin, daß durch eine, vom Operateur leicht zu bewerkstelligende Verbiegung des Instruments im Bereich der Ausströmöffnungen eine Steuerung des Gas-stromes möglich wird, der wiederum einen erheblichen Einfluß auf den Verlauf des Plasmas bzw. Lichtbogens hat.

Darüber hinaus wird durch die Anordnung von mehreren, in Umfangsrichtung des Instruments gesehen winkelmäßig gegeneinander versetzten Ausströmöffnungen selbst bei ungebogener Sonde ein gleichmäßigerer Argonstrom ringsum das Instrument erreicht. Dadurch ist es dem Anwender in einfacherer Weise als bisher möglich, gezielt Läsionen zu behandeln. Diese Vergleichmäßigung des Argonstroms ringsum das Instrument geschieht in bevorzugter Weise dadurch, daß die schlitzförmigen Ausströmöffnungen nach Art eines mehrgängigen Gewindes (insbesondere zwei oder drei "Gewindegänge") rotationssymmetrisch um die Längsachse der Sonde angeordnet sind. Die Elektrodeneinrichtungen sind dementsprechend ebenfalls rotationssymmetrisch zur Sondenlängsachse ausgebildet.

Nachfolgend wird eine bevorzugte Ausführungsform der Erfindung anhand der beiliegenden Abbildungen näher erläutert. Hierbei zeigen:
- Figur 1: eine Ausführungsform des Instruments in schematisierter, perspektivischer Darstellung und
- Figur 2: das Instrument nach Figur 1 im "verbogenen" Zustand.

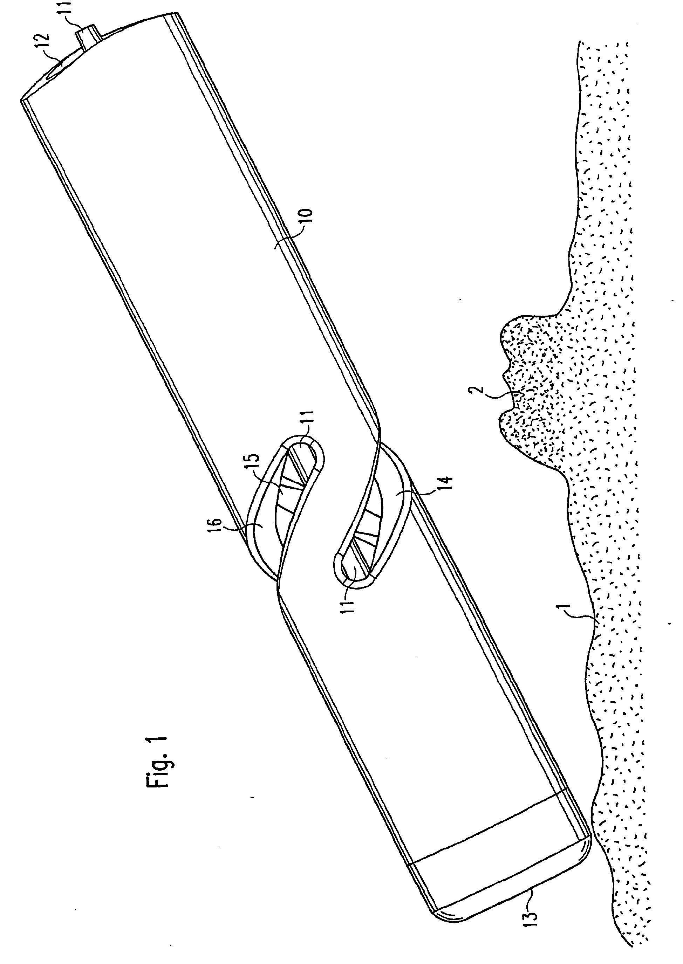

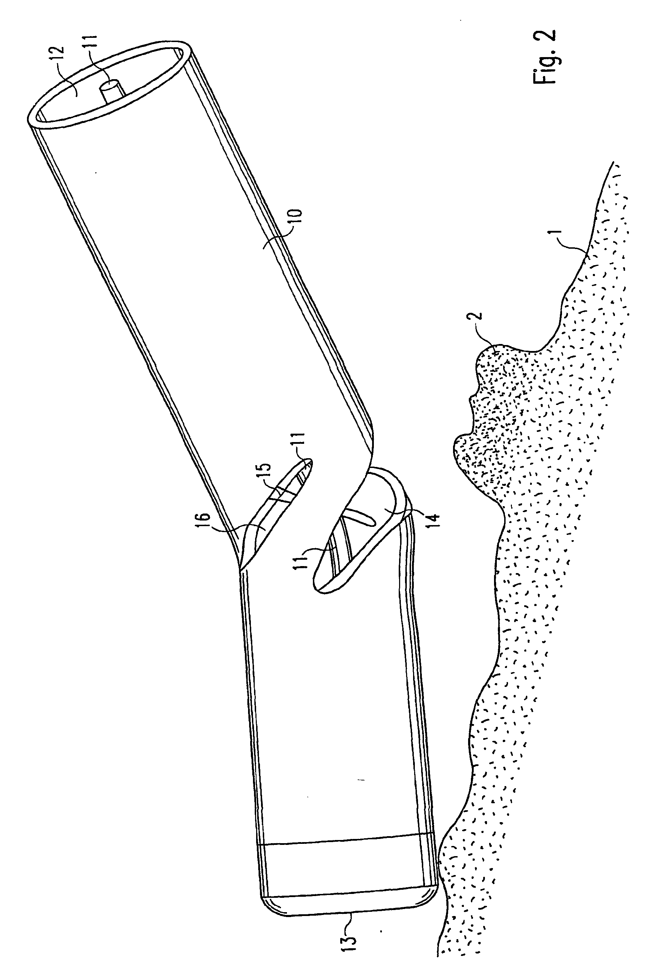

In den Figuren 1 und 2 ist mit der Bezugsziffer 10 ein rohr-oder schlauchförmiges Instrument, insbesondere eine Sonde, insbesondere aus Teflon gezeigt, deren Ende 13 verschlossen ist. In etwa konzentrisch zur Längsachse der Sonde ist in dieser eine Elektrodeneinrichtung, insbesondere ein Draht 11 angeordnet.

Im Endbereich des Instruments 10 sind schraubenförmig, rings um die Umfangsfläche des Instruments 10 und in gleichen Abständen zueinander Ausströmöffnungen 14, 15 und 16 angeordnet. Wird im Betrieb durch ein Lumen 12 des Instruments 10 ein Edelgas, insbesondere Argon oder Helium geleitet, so strömt dies in dem in Figur 1 gezeigten Zustand des Instruments gleichmäßig durch die Ausströmöffnungen 14, 15 und 16 aus, so daß das Instrument 10 in ihrem Endbereich von einer symmetrischen "Argonwolke" umgeben wird. Will man nun eine Läsion 2 auf der Oberfläche eines Gewebes 1 koagulieren, so bringt man das Instrument in die Nähe der Läsion 2, wie dies in Figur 1 gezeigt ist. Dann, wenn die Gefahr besteht, daß das Plasma nicht nur zur Läsion 2 sondern zu Stellen des Gewebes 1 zündet, die insbesondere der Läsion 2 gegenüberliegen (wie dies in engen Körperhöhlen, z.B. im Bronchialsystem der Fall sein kann), so wird das Instrument - wie in Figur 2 gezeigt - mit seinem Ende auf das Gewebe 1 gedrückt. Bei entsprechend elastisch ausgebildetem Material des Instruments 10, insbesondere im Endbereich desselben (die davor liegenden mit der Gas-Quelle verbundenen Abschnitte können steifer ausgebildet sein) verbiegt sich das Instrument, wobei die in Figur 2 mit 15 und 16 bezeichneten Ausströmmöffnungen in ihrem Querschnitt verengt, die der Läsion 2 nächstliegende Ausströmöffnung 14 jedoch erweitert. Dadurch kommt es zu einer automatischen Steuerung des Gasstroms derart, daß ein gezieltes Koagulieren der Läsion 2 ermöglicht wird und das Plasma zwischen der Elektrode 11 und der Läsion 2 bevorzugt gebildet wird. Die Ausströmöffnungen 14, 15 und 16 können so dimensioniert sein, daß nicht nur eine Verengung der einen (15, 16) und eine Erweiterung der anderen (14) Auströmöffnungen, sondern ein vollständiges Schließen einerseits (14, 15) und ein Öffnen der ansich geschlossenen Ausströmöffnung (16) andererseits beim Verbiegen geschieht. Die Ausströmöffnungen 14, 15, und 16 sind in diesem Fall also "Schnitte", deren Flächen im ungebogenen Zustand des Sondenabschnittes aufeinander liegen.

Besonders vorteilhaft ist diese Anordnung dann ausgebildet, wenn die Ausströmöffnungen 14, 15 und 16 absolut symmetrisch, insbesondere um 120° in Umfangsrichtung verteilt in dem Instrument angeordnet sind. Die Länge der Schlitze wird hierbei derart bemessen, daß sich diese mit ihren Enden (im Umfangswinkelrichtung gesehen) sehr nahe sind oder gar überdecken. Dadurch kann es nicht geschehen, daß eine argonfreie Zone in Umfangsrichtung um das Instrument 10 entsteht.

### BEZUGSZEICHENLISTE

- 1: Gewebe
- 2: Läsion
- 10: Instrument
- 11: Elektrode
- 12: Lumen
- 13: verschlossenes Ende
- 14: erste Ausströmöffnung
- 15: zweite Ausströmöffnung
- 16: dritte Ausströmöffnung

## Patentansprüche

1. Elektrochirurgisches Instrument (10) zur Koagulation biologischer Gewebe (1), wobei
aus einer Gasquelle ein Edelgas durch eine rohrförmige Sonde hindurch zu mindestens einer Ausströmöffnung (14,15,16) an einem distalen Endbereich der Sonde zugeführt wird, wobei die Ausströmöffnung eine Längsachse aufweist,
mit einer, im distalen Endbereich der Sonde angeordneten Elektrodeneinrichtung (11), welche mit einer HF-Quelle verbindbar ist, zum Zuführen eines Koagulationsstroms,
wobei die mindestens eine Ausströmöffnung schlitzförmig ausgebildet ist,
**dadurch gekennzeichnet, daß** mindestens zwei einander gegenüberliegende Ausströmöffnungen vorgesehen sind, deren Längsachsen senkrecht oder schräg, jedoch nicht parallel zu einer Längsachse der Sonde verlaufen, und daß die Sonde zumindest im Bereich der Ausströmöffnungen derart biegsam ausgebildet ist, daß bei einem Verbiegen der Sonde im Bereich der Ausströmöffnungen deren Querschnitte vergrößert oder verringert werden.

2. Elektrochirurgisches Instrument nach Anspruch 1,
**dadurch gekennzeichnet, daß**
die Ausströmöffnungen die Sonde schraubenförmig umlaufend nach Art eines mehrgängigen Gewindes ausgebildet sind.

3. Elektrochirurgisches Instrument nach Anspruch 1,
**dadurch gekennzeichnet, daß**
die Ausströmöffnungen im Umfangsrichtung der Sonde gesehen gleich voneinander beabstandet angeordnet sind.

4. Elektrochirurgisches Instrument nach einem der vorhergehenden Ansprüche,
**dadurch gekennzeichnet, daß**
alle schlitzförmigen Ausströmöffnungen dieselbe Länge aufweisen.

5. Elektrochirurgisches Instrument nach einem der vorherigen Ansprüche,
**dadurch gekennzeichnet, daß**
die Enden der Ausströmöffnungen vom Ende der Sonde (13) gleich weit entfernt sind.

6. Elektrochirurgisches Instrument nach einem der vorherigen Ansprüche,
**dadurch gekennzeichnet, daß**
die Ausströmöffnungen derart ausgebildet sind, daß das Edelgas in einem flächen- oder fächerförmigen Strahl austritt.

7. Elektrochirurgisches Instrument nach einem der vorherigen Ansprüche,
**dadurch gekennzeichnet, daß**
die Elektrodeneinrichtungen im Inneren der Sonde derart bei den Ausströmöffnungen angeordnet sind, daß diese jeweils gleiche Abstände zu den Elektrodeneinrichtungen aufweisen.

## Claims

1. Electrosurgical instrument (10) for the coagulation of biological tissue (1),
wherein from a gas source an inert gas is sent through a tubular probe to at least one outflow opening (14, 15, 16) in a distal end region of the probe, such that the outflow opening comprises a longitudinal axis,
with an electrode device (11) which is disposed in the distal end region of the probe and which can be connected to an HF source in order to supply a coagulation current,
such that the at least one outflow opening is/are configured as a slit,
**characterized in that**
at least two outflow openings are provided, disposed opposite one another with their long axes oriented perpendicular to or at an angle to, but not parallel to a long axis of the probe, and that the probe is flexibly constructed at least in the region of the outflow openings, in such a way that when the probe is bent in the region of the outflow openings, their cross sections are enlarged or reduced.

2. Electrosurgical instrument according to Claim 1, **characterized in that** the outflow openings are constructed so as to extend around the probe in a helical configuration, resembling a screw threading with multiple turns.

3. Electrosurgical instrument according to Claim 1, **characterized in that** the outflow openings are uniformly spaced apart from one another, as viewed in the circumferential direction of the probe.

4. Electrosurgical instrument according to one of the preceding claims, **characterized in that** all of the slit-like outflow openings have the same length.

5. Electrosurgical instrument according to one of the preceding claims, **characterized in that** the ends of the outflow openings are equidistant from the end of the probe (13).

6. Electrosurgical instrument according to one of the preceding claims, **characterized in that** the outflow openings are so constructed that the inert gas emerges in a planiform or fan-shaped stream.

7. Electrosurgical instrument according to one of the preceding claims, **characterized in that** the electrode devices in the interior of the probe are disposed next to the outflow openings in such a way that the latter are all at equal distances from the electrode devices.

## Revendications

1. Instrument électrochirurgical (10) pour la coagulation de tissu biologique (1), dans lequel
un gaz rare est acheminé à partir d'une source de gaz à travers une sonde tubulaire vers au moins un orifice de sortie (14, 15, 16) au niveau d'une extrémité distale de la sonde, l'orifice de sortie ayant un axe longitudinal,
comportant un dispositif à électrode (11), qui est agencé dans l'extrémité distale de la sonde et qui peut être relié à une source haute fréquence pour l'alimentation avec un courant de coagulation,
ledit au moins un orifice de sortie étant réalisé sous forme de fente,
**caractérisé en ce qu'**il est prévu au moins deux orifices de sortie face à face, dont les axes longitudinaux sont perpendiculaires ou inclinés, mais pas parallèles, à un axe longitudinal de la sonde, et **en ce que** la sonde, au moins dans la zone des orifices de sortie, est réalisée de manière flexible, de telle sorte qu'une flexion de la sonde dans la zone des orifices de sortie agrandit ou diminue la section desdits orifices.

2. Instrument électrochirurgical selon la revendication 1, **caractérisé en ce que** les orifices de sortie sont réalisés sur le pourtour de la sonde sous forme hélicoïdale, à la manière d'un filetage multiple.

3. Instrument électrochirurgical selon la revendication 1, **caractérisé en ce que** les orifices de sortie sont disposés à égale distance les uns des autres sur la périphérie de la sonde.

4. Instrument électrochirurgical selon l'une quelconque des revendications précédentes, **caractérisé en ce que** tous les orifices de sortie en forme de fente ont la même longueur.

5. Instrument électrochirurgical selon l'une quelconque des revendications précédentes, **caractérisé en ce que** les extrémités des orifices de sortie sont situées à même distance de l'extrémité (13) de la sonde.

6. Instrument électrochirurgical selon l'une quelconque des revendications précédentes, **caractérisé en ce que** les orifices de sortie sont réalisés de telle sorte que le gaz rare sort en formant un faisceau plat ou en éventail.

7. Instrument électrochirurgical selon l'une quelconque des revendications précédentes, **caractérisé en ce que** les dispositifs à électrode sont agencés à l'intérieur de la sonde près des orifices de sortie, de telle sorte que ces derniers sont situés chacun à la même distance des dispositifs à électrode.
